# EUROPEAN PATENT APPLICATION

(11) **EP 3 505 615 A1**
(43) Date of publication of application: **03.07.2019**
(21) Application number: 17843690.3
(22) Date of filing: 24.08.2017
(51) Int. Cl.: C12M 3/00, C12M 1/00

(54) **CELL CULTURE SYSTEM, CULTURE UNIT, AUTOMATIC CELL CULTURE DEVICE, AND CELL CULTURE DEVICE FOR TRANSPORT**

(30) Priority: 26.08.2016 JP 2016166129
(71) Applicant: IHI Corporation, Tokyo 135-8710 (JP)
(72) Inventor: ISHII Kousuke, Tokyo 135-8710 (JP); SAITO Makiko, Tokyo 135-8710 (JP); SAKAI Shin-ichi, Tokyo 135-8710 (JP); YOSHIMURA Akihiko, Tokyo 135-8710 (JP); SHIDA Michinori, Tokyo 135-8710 (JP); NAKAGAWA Masayo, Tokyo 135-8710 (JP)
(74) Representative: Grünecker Patent- und Rechtsanwälte PartG mbB
(86) International application number: PCT/JP2017/030358
(87) International publication number: WO 2018/038214

(57) **Abstract**

In a cell culture system, in a state where a culture unit is contained in a container of an automatic cell culture device, the circulation of a culture solution on a circulation route in the culture unit is controlled by a control unit, and cell culture is performed in a culture tank. At this time, the culture solution is supplied from a culture solution tank, and the cell cultured by a cell collection vial, is collected. In a state where the culture unit is contained in a container of a cell culture device for transport, the circulation of the culture solution on the circulation route in the culture unit is controlled by the control unit, and the cell culture is performed in the culture tank. For this reason, even in a state where the culture unit is attached to the cell culture device for transport, the circulation of the culture solution is controlled, and thus, the culture of the cell is continued.

## Description

### Technical Field

The present disclosure relates to a cell culture system, a culture unit, an automatic cell culture device, and a cell culture device for transport. This application is based upon and claims the benefit of priority from Japanese Patent Application No. 2016-166129, filed on Aug. 26, 2016 and claims the benefit of priority thereto; the entire contents of which are incorporated herein by reference.

### Background Art

For example, in Patent Literature 1, a cell culture device including a cell culture unit performing three-dimensional culture, a liquid feeding unit feeding various medicinal solutions, a culture solution, a waste solution, and the like, is disclosed as a cell culture device. The cell culture device is a device for culturing a cell on a suspended carrier in the culture solution, and is provided with a control unit controlling each unit, in order to automatically perform an operation such as medium replacement and passage, which was manually performed by an operator in the related art.

### Citation List

### Patent Literature

Patent Literature 1: Japanese Unexamined Patent Publication No. 2015-142550

### Summary of Invention

### Technical Problem

However, recently, the cell culture system has been required to efficiently perform the culture, according to the expansion of application with respect to the medical field. In addition, there is a case where a location where the cell culture is performed, is different from a location where the cultured cell is used, but the transport of the cultured cell has not been sufficiently considered.

In the present disclosure, a cell culture system capable of performing transport with suppressing the degradation in quality, while efficiently performing cell culture, and a culture unit, an automatic cell culture device, and a cell culture device for transport, provided in the cell culture system, will be described.

### Solution to Problem

A cell culture system according to one aspect of the present disclosure, is a cell culture system including: a culture unit including a culture tank for culturing a cell in a culture solution; an automatic cell culture device automatically controlling the culture of the cell in the culture unit; and a cell culture device for transport, controlling the culture of the cell in the culture unit at the time of transporting the culture unit, in which the culture unit includes the culture tank in a shape in which a horizontal sectional area upwardly increases, and a circulation route for circulating the culture solution such that the culture solution forms an upward flow in the culture tank, the automatic cell culture device includes a first container capable of containing the culture unit, a first culture environment adjustment unit including a first culture solution supply unit supplying the culture solution to the culture tank of the culture unit contained in the first container, and a first cell collection unit collecting the cell in the culture tank, and adjusting a culture environment in the culture tank of the culture unit contained in the first container, and a first control unit controlling the circulation of the culture solution along the circulation route of the culture unit, and controlling the adjustment of the culture environment of the first culture environment adjustment unit, and the cell culture device for transport includes a second container capable of containing the culture unit, and a second control unit controlling the circulation of the culture solution along the circulation route of the culture unit contained in the second container.

### Effects of Invention

According to the present disclosure, it is possible to perform transport with suppressing the degradation in quality, while efficiently performing cell culture.

### Brief Description of Drawings

FIG. 1 is a diagram illustrating a schematic configuration of an automatic cell culture device of a cell culture system.
FIG. 2 is a diagram illustrating a schematic configuration of a cell culture device for transport of the cell culture system.
FIG. 3 is a diagram illustrating the automatic cell culture device and a culture unit of the cell culture system.
FIG. 4 is a diagram illustrating a movement of a culture solution in a culture tank of the culture unit.
FIG. 5 is a diagram illustrating the cell culture device for transport and the culture unit of the cell culture system.
FIG. 6 is a diagram illustrating a modification example of the culture unit.
FIG. 7 is a diagram illustrating a modification example of the culture unit.

### Description of Embodiments

A cell culture system according to one aspect of the present disclosure, is a cell culture system including: a culture unit including a culture tank for culturing a cell in a culture solution; an automatic cell culture device automatically controlling the culture of the cell in the culture unit; and a cell culture device for transport, controlling the culture of the cell in the culture unit at the time of transporting the culture unit, in which the culture unit includes the culture tank in a shape in which a horizontal sectional area upwardly increases, and a circulation route for circulating the culture solution such that the culture solution forms an upward flow in the culture tank, the automatic cell culture device includes a first container capable of containing the culture unit, a first culture environment adjustment unit including a first culture solution supply unit supplying the culture solution to the culture tank of the culture unit contained in the first container, and a first cell collection unit collecting the cell in the culture tank, and adjusting a culture environment in the culture tank of the culture unit contained in the first container, and a first control unit controlling the circulation of the culture solution along the circulation route of the culture unit, and controlling the adjustment of the culture environment of the first culture environment adjustment unit, and the cell culture device for transport includes a second container capable of containing the culture unit, and a second control unit controlling the circulation of the culture solution along the circulation route of the culture unit contained in the second container.

In addition, a culture unit according to one aspect of the present disclosure, is a culture unit provided in the cell culture system described above, the unit including: the culture tank in a shape in which a horizontal sectional area upwardly increases; and a circulation route for circulating the culture solution such that the culture solution forms an upward flow in the culture tank, in which the culture unit is removable with respect to the automatic cell culture device and the cell culture device for transport.

In addition, an automatic cell culture device according to one aspect of the present disclosure, is an automatic cell culture device provided in the cell culture system including a culture unit including a culture tank for culturing a cell in a culture solution, an automatic cell culture device automatically controlling the culture of the cell in the culture unit, and a cell culture device for transport, controlling the culture of the cell in the culture unit at the time of transporting the culture unit, the device including: a first container capable of containing the culture unit including the culture tank in a shape in which a horizontal sectional area upwardly increases, and a circulation route for circulating the culture solution such that the culture solution forms an upward flow in the culture tank; a first culture environment adjustment unit including a first culture solution supply unit supplying the culture solution to the culture tank of the culture unit contained in the first container, and a first cell collection unit collecting the cell in the culture tank, and adjusting a culture environment in the culture tank of the culture unit contained in the first container; and a first control unit controlling the circulation of the culture solution along the circulation route of the culture unit contained in the first container, and controlling the adjustment of the culture environment of the first culture environment adjustment unit.

In addition, a cell culture device for transport according to one aspect of the present disclosure, is a cell culture device provided in the cell culture system including a culture unit including a culture tank for culturing a cell in a culture solution, an automatic cell culture device automatically controlling the culture of the cell in the culture unit, and a cell culture device for transport, controlling the culture of the cell in the culture unit at the time of transporting the culture unit, the device including: a second container capable of containing the culture unit including the culture tank in a shape in which a horizontal sectional area upwardly increases, and a circulation route for circulating the culture solution such that the culture solution forms an upward flow in the culture tank; and a second control unit controlling the circulation of the culture solution along the circulation route of the culture unit contained in the second container.

According to the cell culture system, and the culture unit, the automatic cell culture device, and the cell culture device for transport, provided in the cell culture system, in a state where the culture unit is contained in the first container of the automatic cell culture device, the circulation the of the culture solution on the circulation route in the culture unit is controlled by the first control unit, and thus, the culture of the cell is performed in a state where the upward flow of the culture solution is formed in the culture tank. At this time, the culture solution is supplied from the first culture solution supply unit of the first culture environment adjustment unit, and the cell cultured by the first cell collection unit, is collected, and thus, it is possible to automatically perform the cell culture. On the other hand, in a state where the culture unit is contained in the second container of the cell culture device for transport, the circulation of the culture solution on the circulation route in the culture unit is controlled by the second control unit, and thus, the culture of the cell is performed in a state where the upward flow of the culture solution is formed in the culture tank. For this reason, even in a state where the culture unit is attached to the cell culture device for transport, the circulation of the culture solution is controlled, and the culture of the cell is continued. Therefore, in the automatic cell culture device, it is possible to efficiently perform the cell culture, and it is possible to perform the transport with suppressing the degradation in quality by the cell culture device for transport, at the time of performing the transport.

Here, the culture unit is capable of including identification information for specifying the culture unit, which is individually applied, a storage unit storing information relevant to the culture of the cell in the culture unit, in association with the identification information, can be further provided, and the first control unit of the automatic cell culture device and the second control unit of the cell culture device for transport are capable of respectively acquiring the information relevant to the culture of the cell in the culture unit, from the storage unit, on the basis of the identification information, and performing control relevant to the culture unit, on the basis of the information.

According to the configuration described above, the identification information for specifying the culture unit is individually applied, and the information relevant to the cell culture is stored in the storage unit, in association with the identification information. Therefore, in the first control unit of the automatic cell culture device and the second control unit of the cell culture device for transport, it is possible to acquire the information relevant to the cell culture in the culture unit from the storage unit by using the identification information, and to perform control using the information, and thus, it is possible to more suitably perform the control relevant to the cell culture of the culture unit, for each culture unit.

In addition, the culture unit is capable of further including a vibration suppression unit for suppressing a vibration of the culture tank.

As described above, in the case of including the vibration suppression unit for suppressing the vibration of the culture tank of the culture unit, it is possible to prevent the vibration from affecting the cell culture in the culture tank, at the time of performing the transport by attaching the culture unit to the cell culture device for transport.

In addition, the automatic cell culture device is capable of further including a first cell distribution detection unit detecting a distribution of the cell in the culture tank of the culture unit contained in the first container, and the first control unit is capable of controlling a supply amount per unit time of the culture solution to be supplied to the culture tank by the first culture solution supply unit, on the basis of the distribution of the cell in the culture tank, detected by the first cell distribution detection unit.

As described above, the supply amount per unit time of the culture solution to be supplied to the culture tank, is controlled on the basis of the distribution of the cell in the culture tank, detected by the first cell distribution detection unit, and thus, in a case where the distribution of the cell in the culture tank is changed from the assumed situation, it is possible to automatically control the supply amount per unit time of the culture solution in order to correct the change. Therefore, it is possible to more preferably control the supply amount of the culture solution according to the growth of the cell, while stabilizing the flow of the culture solution in the culture tank.

In addition, the automatic cell culture device is capable of further including a first culture environment detection unit detecting the culture environment in the culture tank of the culture unit contained in the first container, and the first control unit is capable of controlling the adjustment of the culture environment of the first culture environment adjustment unit, on the basis of the culture environment in the culture tank, detected by the first culture environment detection unit.

As described above, the adjustment of the first culture environment adjustment unit adjusting the culture solution to be supplied to the culture tank, is controlled on the basis of the culture environment in the culture tank, detected by the first culture environment detection unit, and thus, it is possible to automatically control the adjustment for improving the culture environment in the culture tank. Therefore, it is possible to preferably maintain the culture environment according to the growth of the cell in the culture tank.

In addition, the cell culture device for transport is capable of further including a second culture environment adjustment unit including a second culture solution supply unit supplying the culture solution to the culture tank of the culture unit contained in the second container, and a second cell collection unit collecting the cell in the culture tank, and the second control unit is capable of controlling the second culture environment adjustment unit.

As described above, in a case where the cell culture device for transport includes the second culture environment adjustment unit including the second culture solution supply unit and the second cell collection unit, it is possible to prevent the culture solution from being degraded due to the prolonged culture period, and to preferably perform the collection of the cultured cell, and the like, even in a case where the transport of the culture unit including the culture tank is prolonged by using the cell culture device for transport. Therefore, it is possible to preferably perform the transport of the culture unit, using the cell culture device for transport.

In addition, the cell culture device for transport is capable of further including a second cell distribution detection unit detecting a distribution of the cell in the culture tank of the culture unit contained in the second container, and the second control unit is capable of controlling a supply amount per unit time of the culture solution to be supplied to the culture tank by the second culture solution supply unit, on the basis of the distribution of the cell in the culture tank, detected by the second cell distribution detection unit.

As described above, the supply amount per unit time of the culture solution to be supplied to the culture tank, is controlled on the basis of the distribution of the cell in the culture tank, detected by the first cell distribution detection unit, and thus, in a case where the distribution of the cell in the culture tank is changed from the assumed situation, it is possible to automatically control the supply amount per unit time of the culture solution in order to correct the change. Therefore, it is possible to more preferably control the supply amount of the culture solution according to the growth of the cell, while stabilizing the flow of the culture solution in the culture tank.

In addition, the cell culture device for transport is capable of further including a second culture environment detection unit detecting the culture environment in the culture tank of the culture unit contained in the second container, and the second control unit is capable of controlling the adjustment of the culture environment of the second culture environment adjustment unit, on the basis of the culture environment in the culture tank, detected by the second culture environment detection unit.

As described above, the adjustment of the second culture environment adjustment unit adjusting the culture solution to be supplied to the culture tank, is controlled on the basis of the culture environment in the culture tank, detected by the second culture environment detection unit, and thus, it is possible to automatically control the adjustment for improving the culture environment in the culture tank. Therefore, it is possible to preferably maintain the culture environment according to the growth of the cell in the culture tank.

Hereinafter, a mode for carrying out the present disclosure will be described in detail, with reference to the attached drawings. Furthermore, the same reference numerals will be applied to the same constituents in the description of the drawings, and the repeated description will be omitted.

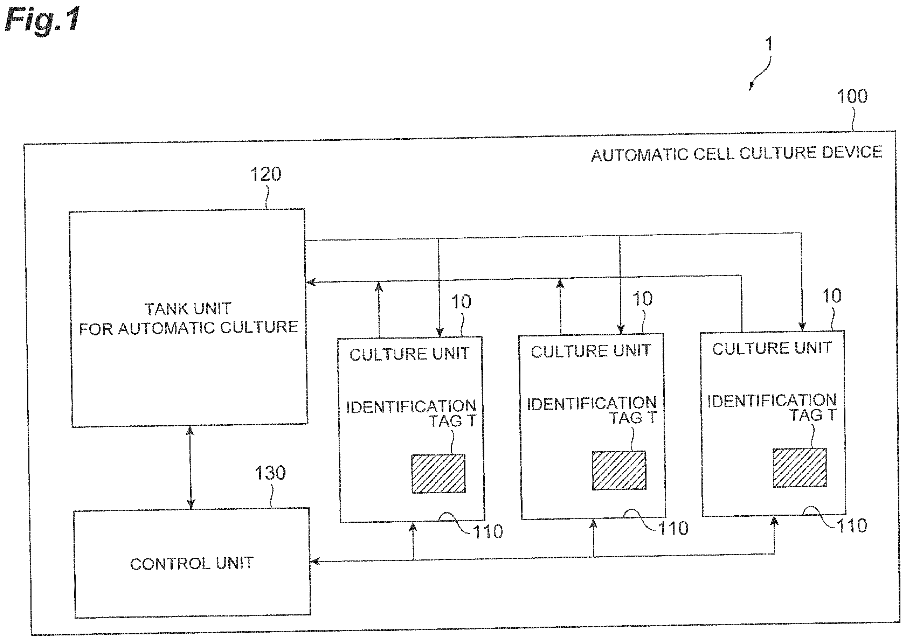

FIG. 1 to FIG. 5 are diagrams illustrating a schematic configuration of each device configuring a cell culture system according to one aspect of the present disclosure. Hereinafter, a schematic configuration of the cell culture system will be described, and then, an automatic cell culture device, a culture unit, and a cell culture device for transport, provided in the cell culture system, will be described.

### (Schematic Configuration of Cell Culture System)

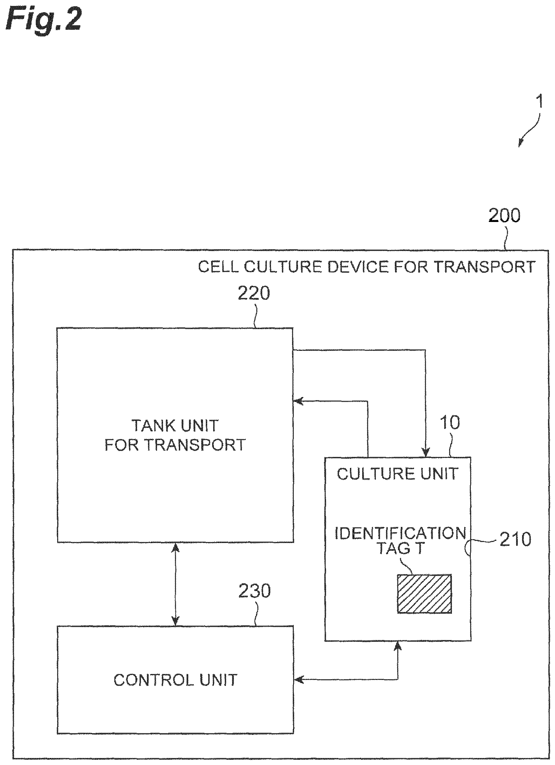

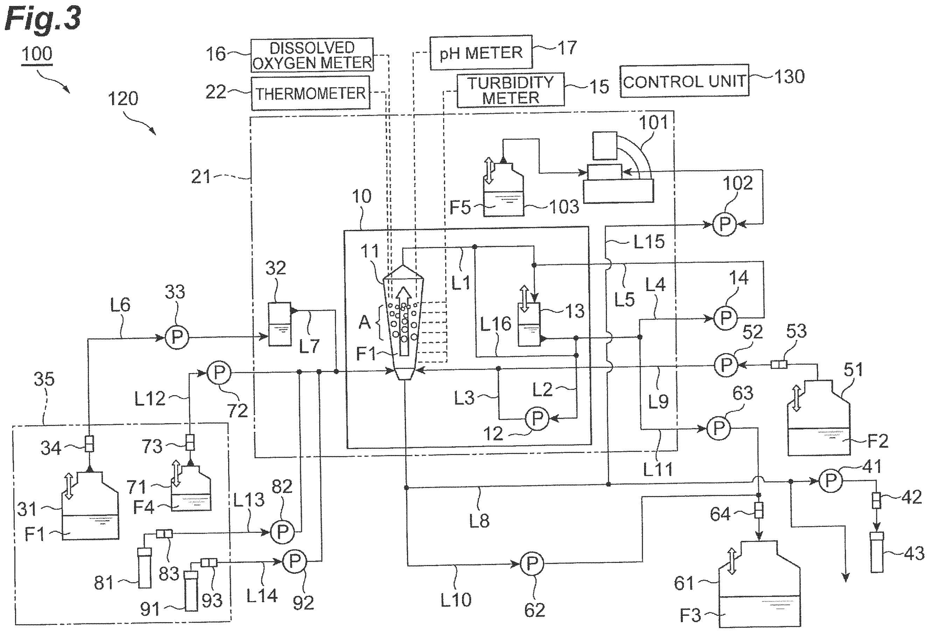

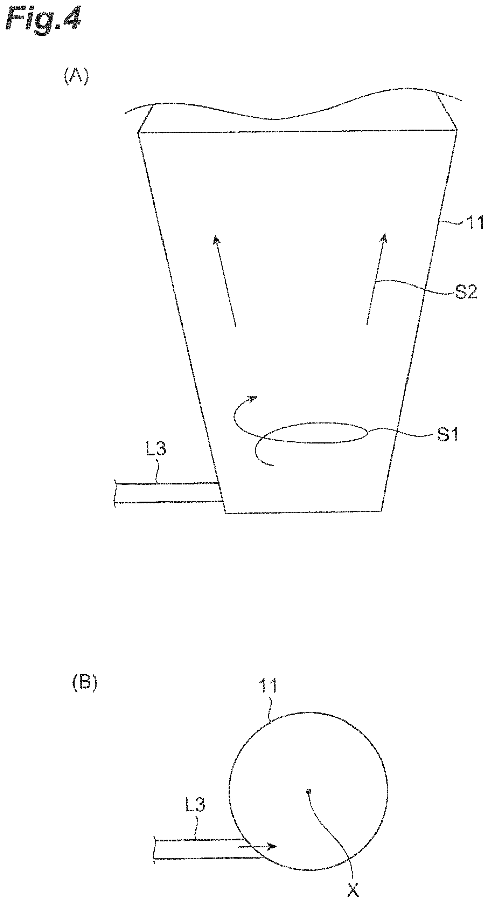

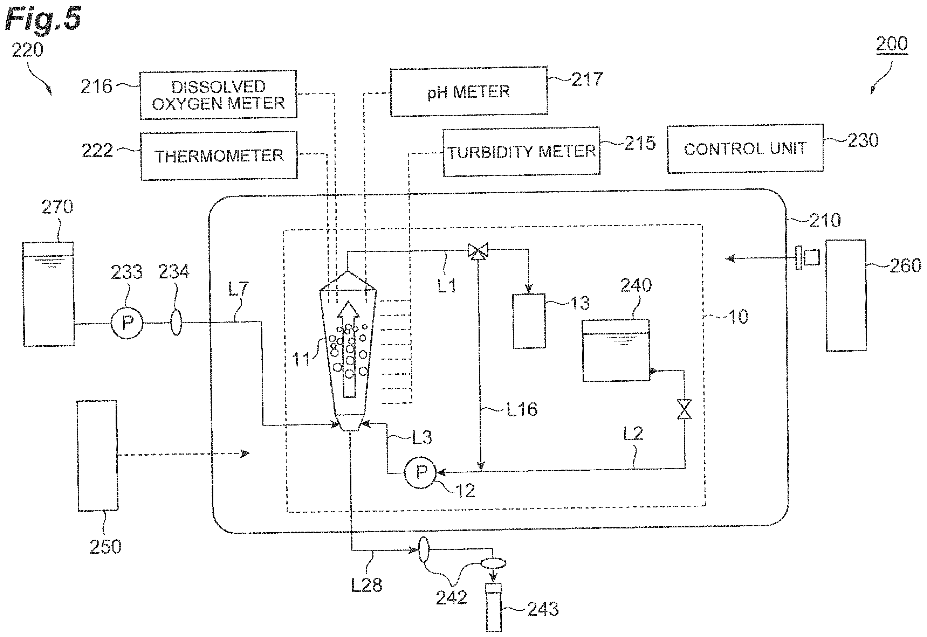

A cell culture system 1 according to this embodiment, includes a culture unit 10 illustrated in FIG. 1, an automatic cell culture device 100 automatically control the culture of a cell in the culture unit, and a cell culture device 200 for transport, controlling the culture of the cell in the culture unit at the time of transporting the culture unit, illustrated in FIG. 2. In addition, FIG. 3 is a diagram illustrating the detailed configuration of the automatic cell culture device 100 and the culture unit 10, and FIG. 4 is a diagram illustrating a culture tank. In addition, FIG. 5 is a diagram illustrating the detailed configuration of the cell culture device 200 for transport.

The automatic cell culture device 100 illustrated in FIG. 1, one or more containers 110 (first containers) for containing includes one or more culture units 10, a tank unit 120 for automatic culture (a first culture environment adjustment unit) connected to each of one or more culture units 10 attached to the containers 110, and a control unit 130 (a first control unit). The details of the culture unit 10 will be described below, and the culture unit 10 includes one culture tank for culturing the cell and a reserving tank reserving a culture solution that is circulated in the culture tank. The culture tank has a shape in which a sectional area in a horizontal direction upwardly increases, and an upward flow of the culture solution is formed in the culture tank, and thus, the culture is performed in a state where the cell is retained in a predetermined region in the culture tank.

The culture unit 10 including the culture tank, is configured as a unit removable from the automatic cell culture device 100, and the removed culture unit 10 is attached to the cell culture device 200 for transport, illustrated in FIG. 2, and thus, it is possible to perform the transport in culture tank unit, in a state where the culture of the cell in the culture tank is continued.

In an example of the automatic cell culture device 100 illustrated in FIG. 1, the culture unit 10 is contained in each of three containers 110. An identification tag T is attached to each of the culture units 10. The identification tag T, for example, is realized by an IC tag, a QR code (Registered Trademark), or the like. The culture unit 10, as described above, is removable with respect to the automatic cell culture device 100 and the cell culture device 200 for transport. Therefore, in the identification tag T1, information relevant to the cell that is cultured by the culture unit 10, is associated with information for specifying the culture unit 10.

The tank unit 120 for automatic culture of the automatic cell culture device 100 indicates tanks or pumps for performing the culture of the cell in the culture tank of the culture unit 10 in the automatic cell culture device 100. The details thereof will be described below, and for example, a tank of the culture solution to be supplied to the culture tank, a tank of a medicinal agent to be added to the culture solution, a pump for supplying the culture solution and the medicinal agent to the culture tank, and the like are included in the tank unit 120 for automatic culture. Each of the tanks and the like included in the tank unit 120 for automatic culture, and the culture unit 10 are connected to each other through a line (pipe arrangement or a pipe line) or the like.

The control unit 130 has a function of controlling each unit included in the culture unit 10 contained in a container 110, and each unit included in the tank unit 120 for automatic culture, and of controlling automatic cell culture according to the culture unit 10 attached to the automatic cell culture device 100. The details will be described below.

The cell culture device 200 for transport illustrated in FIG. 2, includes a container 210 (a second container) for containing one culture unit 10, a tank unit 220 for transport (a second culture environment adjustment unit) that is connected to the culture unit 10 attached to the container 210, and a control unit 230 (a second control unit).

The tank unit 220 for transport of the cell culture device 200 for transport indicates tanks or pumps for performing the culture of the cell in the culture tank of the culture unit 10 in the cell culture device 200 for transport. The details will be described below, and for example, a tank of the culture solution to be supplied to the culture tank, a pump for supplying the culture solution to the culture tank, and the like are included in the tank unit 220 for transport. Furthermore, in the tank unit 220 for transport of the cell culture device 200 for transport, the tank or the like is downsized and the number of tanks or the like is also reduced, compared to the tank unit 120 for automatic culture of the automatic cell culture device 100. This is because in the tank unit 220 for transport of the cell culture device 200 for transport, a tank required for attaining the transport of the culture tank while continuing the culture of the cell, is provided by being selected.

The control unit 230 has a function of controlling each unit included in the culture unit 10 contained in the container 210, and each unit included in the tank unit 220 for transport, and of controlling the cell culture of the culture unit 10 attached to the cell culture device 200 for transport.

The control unit 130 of the automatic cell culture device 100 and the control unit 230 of the cell culture device 200 for transport are respectively configured as a computer provided with hardware such as a central processing unit (CPU), a random access memory (RAM) and a read only memory (ROM) that are a main storage device, a communication module performing communication with respect to other devices, and an auxiliary storage device such as a hard disk. Then, such constituents are operated, and thus, each function is exhibited.

### (Automatic Cell Culture Device and Culture Unit)

Next, the automatic cell culture device 100 and the culture unit 10 will be described with reference to FIG. 3. In FIG. 3, a state in which the culture unit 10 is contained in the container 110 of the automatic cell culture device 100, and the culture unit 10 and each unit of the tank unit 120 for automatic culture in the automatic cell culture device 100 are connected to each other through the line, is illustrated. In addition, in FIG. 3, the control unit 130 of the automatic cell culture device 100 is also illustrated. That is, in each of the units included in the automatic cell culture device 100 illustrated in the FIG. 3, each of the units except for the control unit 130, is included in the tank unit 120 for automatic culture, and corresponds to the first culture environment adjustment unit of the automatic cell culture device 100.

As illustrated in FIG. 3, the culture unit 10 includes a culture tank 11 of performing three-dimensional culture of the cell suspended in a culture solution F1, a circulation pump 12 for circulating the culture solution F1 such that the culture solution F1 forms a circulation upward flow in the culture tank, and an oxygen supply tank 13 supplying oxygen to the culture solution F1, which is provided in the middle of the circulation of the culture solution F1. In the culture unit 10, a circulation route of the culture solution is configured by a line connecting the culture tank 11, the circulation pump 12, and the oxygen supply tank 13 to each other. The culture tank 11 and the oxygen supply tank 13 are connected to each other through a first circulation line L1, and the culture solution F1 extracted from an upper end portion of the culture tank 11, is fed to the oxygen supply tank 13 through the first circulation line L1. The oxygen supply tank 13 and the circulation pump 12 are connected to each other through a second circulation line L2, and the culture solution F1 to which oxygen is supplied as necessary in the oxygen supply tank 13, is fed to the circulation pump 12 through the second circulation line L2. The circulation pump 12 and the culture tank 11 are connected to each other through a third circulation line L3, the culture solution F1 fed from the circulation pump 12 is supplied to a lower end of the culture tank 11 through the third circulation line L3. The culture solution F1 supplied through the third circulation line L3, is moved in the circulation upward flow from the lower side to the upper side of the culture tank 11, and is fed from the first circulation line L1 to the outside of the culture tank 11.

The culture tank 11 included in the culture unit 10, will be further described with reference to FIG. 4. The culture tank 11 has a shape in which the sectional area in the horizontal direction upwardly increases. For example, the culture tank 11 can be in an inverted conical shape, as the shape described above, but the shape of the culture tank 11 is not limited to the inverted conical shape. The culture tank 11 is filled with the culture solution F1. In addition, in the culture tank 11, a culture region A is formed in which a cell or a carrier to which the cell is attached float in a predetermined height range. Hereinafter, the cell or the carrier to which the cell is attached, will be referred to as a culture cell.

The content of the culture tank 11 is not particularly limited, but can be approximately 10 mL to 10,000 mL. The content of the culture tank 11 can be suitably selected on the basis of the amount of cell to be cultured in one culture tank 11, the size of a cell mass, or the like. In addition, the content of the culture tank 11 can be selected according to handling properties of the culture tank 11, or the like. For example, in a case where the content of the culture tank 2 is approximately 50 mL to 500 mL, the handling properties as the single culture tank 11 or the culture unit 10, are improved.

The third circulation line L3 is connected to the lower end of the culture tank 11. In addition, the first circulation line L1 is connected to an upper end of the culture tank 11.

In the culture tank 11, the upward flow of the culture solution F1 is formed. FIG. 4(A) is a diagram illustrating the flow of the culture solution F1, which is generated in the culture tank 11, and FIG. 4(B) is a sectional view of the culture tank 11 in the horizontal direction, and is a diagram illustrating an attachment position of the third circulation line L3 with respect to the culture tank 11.

As illustrated in FIG. 4(B), the third circulation line L3 is attached to the culture tank 11, in a position not intersecting with a central axis X extending in an up-down direction in the culture tank 11. That is, the central axis X and the third circulation line L3 are twisted with each other. In such a state, in a case where the culture solution is supplied from the third circulation line L3 into the culture tank 2, the culture solution is moved up while being rotated along an inner wall of the culture tank 11 by a turning force generated due to friction between the culture solution and the wall surface in the culture tank 11. That is, as illustrated in FIG. 4(A), a turning upward flow S1 that is one type of circulation upward flow, is generated from the lower side to the upper side of the culture tank 11. In this embodiment, the circulation upward flow indicates that a liquid in a tank is moved up while being circulated along an inner wall of the tank.

The turning force due to the friction with respect to the wall surface of the culture tank 11 decreases, as the turning upward flow S1 of the culture solution in the culture tank 11 is moved up, and thus, as illustrated in FIG. 4(A), a upward flow S2 is formed in which the moving-up is more mainly performed than the turning. Accordingly, in the vicinity of the culture region A of the culture tank 11 (refer to FIG. 3), a flow (a plug flow) having an approximately constant flow rate distribution in a moving-up direction on a horizontal surface, is formed.

In the culture tank 11, the horizontal sectional area upwardly increases. Therefore, a flow rate of the upward flow S2 in the plug flow decreases as being directed towards the upper side of the culture tank 11. In the vicinity of the culture region A of the culture tank 11, a state is formed in which a sedimentation rate of the culture cell and the flow rate of the upward flow S2 are balanced. As a result thereof, the culture cells are respectively retained in a predetermined height position. In addition, a shear stress due to the upward flow S1 acts on the culture cell, and thus, the cell that has excessively grown, is crushed, and it is possible to maintain the entire cell diameter. Furthermore, the circulation upward flow can also be formed in the culture tank by a method different from the adjustment of the attachment position of the third circulation line L3, described above. A method of forming the circulation upward flow is not limited to the example described above.

Thus, in the culture tank 11, the culture solution F1 is circulated through the first circulation line L1 to the third circulation line L3, and thus, the culture of the cell progresses.

Furthermore, the shape of a vessel, the shape of the upward flow, or the like is not limited to the above description, insofar as the culture tank 11 of the culture unit 10 of a cell culture system 1 according to this embodiment, has a shape in which the horizontal sectional area upwardly increases, and the culture of the cell is performed in the culture solution where the upward flow is formed.

Returning to FIG. 3, in the automatic cell culture device 100, a turbidity meter 15 measuring the turbidity of the culture solution F1 in the culture tank 11 in the culture unit 10 in a plurality of spots in a vertical direction, is provided. In the automatic cell culture device 100, the cell distribution in the culture solution F1 in the culture tank 11 is detected by the turbidity meter 15. Furthermore, the automatic cell culture device 100 includes the turbidity meter 15 as the first cell distribution detection unit, but the first cell distribution detection unit is not limited thereto, and for example, may include a camera acquiring an image of the culture solution F1, or the like.

In the automatic cell culture device 100, a microbubble pump 14 is provided as an auxiliary means aiding the supply of oxygen with respect to the culture solution F1 in the oxygen supply tank 13. The microbubble pump 14 is connected to the oxygen supply tank 13 through a fourth circulation line L4 branching off from the second circulation line L2, and a fifth circulation line L5 connected to the first circulation line L1. A part of the culture solution F1 passing through the second circulation line L2, is fed to the microbubble pump 14 through the fourth circulation line L4, and is returned to the oxygen supply tank 13 from the microbubble pump 14 through the fifth circulation line L5 and the first circulation line L1. The microbubble pump 14 generates microbubbles in the culture solution F1, and accelerates the dissolution of oxygen with respect to the culture solution F1. In addition, the automatic cell culture device 100 includes a dissolved oxygen meter 16 detecting the amount of dissolved oxygen in the culture solution F1 in the culture tank 11. The dissolved oxygen meter 16 may detect the amount of dissolved oxygen before the culture is started, may constantly detect the amount of dissolved oxygen during the culture, or may detect the amount of dissolved oxygen at a predetermined interval during the culture.

The automatic cell culture device 100 further includes a pH meter 17 detecting the pH of the culture solution F1 in the culture tank 11. The pH meter 17 may detect the pH before the culture is started, may constantly detect the pH during the culture, or may detect the Ph at a predetermined interval during the culture. A pH detection result of the pH meter can be a determination standard of a change-out rate of the culture solution F1.

The culture tank 11 and the first circulation line L1 to the third circulation line L3 are arranged in an incubator 21 for retaining a culture environment in the culture tank 11 to be constant. That is, in the automatic cell culture device 100, the container 110 is provided in the incubator 21, and the culture unit 10 including the culture tank 11 and the first circulation line L1 to the third circulation line L3 is contained in the incubator 21. The incubator 21 adjusts and maintains the inside to a predetermined culture environment (for example, a temperature of 35°C to 38°C, and a carbon dioxide concentration of 5%), and prevent the culture environment from varying due to the external environment. In the automatic cell culture device 100, in order to avoid a temperature change in the culture solution F1 at the time of supplying oxygen, the oxygen supply tank 13 is also provided in the incubator 21. The automatic cell culture device 100 may include a thermometer 22 measuring the temperature in the culture tank 11 or the temperature of the culture solution F1 in the culture tank 11, and in such a case, it is possible to confirm that the inside of the culture tank 11 is adjusted to a predetermined culture environment by the thermometer 22, and is maintained. Furthermore, in the automatic cell culture device 100, the dissolved oxygen meter 16, the pH meter 17, and the thermometer 22 function as the first culture environment detection unit detecting the culture environment, and may include a means detecting the culture environment, in addition to the above description.

Furthermore, the first circulation line L1 to the third circulation line L3 through which the culture solution F1 of the culture tank 11 is circulated, are provided in the incubator 21. Accordingly, the culture solution F1 circulated in the culture tank 11 is maintained to a predetermined culture temperature.

The automatic cell culture device 100 further includes a culture solution storage tank 31 storing the culture solution F1, a culture solution heating tank 32 heating the culture solution F1, and a culture solution feed line L6 connecting the culture solution storage tank 31 and the culture solution heating tank 32 together, and a culture solution feeding pump 33 is provided in the culture solution feed line L6. Each of the units described above, functions as the first culture solution supply unit in the automatic cell culture device 100. The culture solution F1 stored in the culture solution storage tank 31, as necessary, is fed to the culture solution heating tank 32 through the culture solution feed line L6, and is heated in the culture solution heating tank 32 to a temperature suitable for the culture. The culture solution F1 heated in the culture solution heating tank 32, is fed to the culture tank 11 from the culture solution heating tank 32 through the heated culture solution feed line L7 connecting the culture solution heating tank 32 and the culture tank 11 together. The culture solution storage tank 31 and the culture solution feed line L6 are connected to each other through a sterile connector 34. Accordingly, it is possible to easily maintain a sterile state after the culture solution feed line L6, at the time of replacing the culture solution storage tank 31. The culture solution storage tank 31 is provided in a refrigerating unit 35. The refrigerating unit 35 retains the culture solution storage tank 31 at a temperature suitable for the storage of the culture solution F1 (for example, 2°C to 6°C). The culture solution heating tank 32 is provided in the incubator 21, and the incubator 21 heats the culture solution F1 in the culture solution heating tank 32 to a temperature suitable for the culture (for example, 35°C to 38°C).

The automatic cell culture device 100 further includes a cell collection line L8 that is a collection route for collecting the cultured cell from the culture tank 11, and a cell collection pump 41 is provided in the cell collection line L8. A sterile connector 42 is provided in an outlet of the cell collection line L8, and thus, the cell collection line L8 can be sterilely connected to a cell collection vial 43. In the automatic cell culture device 100, the cell collection vial 43 is connected to the sterile connector 42, and then, the cell is circulated through the cell collection line L8 from the culture tank 11, according to the operation of the cell collection pump 41, and thus, the cultured cell can be collected. Each of the units described above functions as the first cell collection unit in the automatic cell culture device 100.

The automatic cell culture device 100 further includes a wash solution storage tank 51 storing a wash solution F2, and a wash solution feeding pump 52 feeding the wash solution F2 to the culture tank 11 from the wash solution storage tank 51. The wash solution storage tank 51 is connected to the culture tank 11 through a wash solution feed line L9, and the wash solution F2 stored in the wash solution storage tank 51, as necessary, is fed to the culture tank 11 through the wash solution feed line L9, by the wash solution feeding pump 52. The wash solution feed line L9 is connected to the third circulation line L3 that is the circulation route of the culture unit, and the wash solution F2 is fed to the culture tank 11 through the wash solution feed line L9 and the third circulation line L3, and is circulated on the circulation route of the culture unit according to a case, and then, is discharged from the culture unit as a waste solution. The wash solution storage tank 51 and the wash solution feed line L9 are connected to each other through a sterile connector 53. Accordingly, it is possible to easily maintain a sterile state after the wash solution feed line L9, at the time of replacing the wash solution storage tank 51.

The wash solution F2 is not particularly limited, and may be a known wash solution used in the cell culture. For example, the wash solution F2 may be phosphate buffered saline (PBS). In addition, the wash solution F2 may be a basal medium suitable for the cell to be cultured, or the like. For example, DMEM, F-12, RPMI, or the like is a representative medium.

The automatic cell culture device 100 further includes a waste solution reserving tank 61 reserving a waste solution F3 collected from the culture unit 10. In addition, the automatic cell culture device 100 includes first waste solution line L10 connecting the culture tank 11 and the waste solution reserving tank 61 together, as a first waste solution route, and a second waste solution line L11 connecting the oxygen supply tank 13 and the waste solution reserving tank 61 together, as a second waste solution route, respectively. A first waste solution pump 62 feeding the waste solution to be discharged from the culture tank 11, to the waste solution reserving tank 61, is provided in the first waste solution line L10, and a second waste solution pump 63 feeding the waste solution to be discharged from the oxygen supply tank 13, to the waste solution reserving tank 61, is provided in the second waste solution line L11. The first waste solution line L10 and the second waste solution line L11 are join together before reaching the waste solution reserving tank 61, and are connected to the waste solution reserving tank 61 through a sterile connector 64. It is possible to replace the waste solution reserving tank 61 while easily maintaining a sterile state of the first waste solution line L10 and the second waste solution line L11 by the sterile connector 64. The waste solution reserved in the waste solution reserving tank 61, may be a waste solution including the culture solution or the wash solution, and for example, may be the used culture solution at the time of replacing the culture solution, the used culture solution to be discarded before the cell collection or the passage, a waste solution generated after performing the washing with the wash solution, or the like.

The automatic cell culture device 100 further includes an enzyme solution storage tank 71 storing an enzyme solution F4 containing an enzyme, and an enzyme solution feeding pump 72 feeding the enzyme solution F4 to the culture tank 11 from the enzyme solution storage tank 71. The enzyme solution storage tank 71 is connected to the culture tank 11 through an enzyme solution feed line L12, and the enzyme solution F4 stored in the enzyme solution storage tank 71, as necessary, is fed to the culture tank 11 through the enzyme solution feed line L12, by the enzyme solution feeding pump 72. The enzyme solution storage tank 71 and the enzyme solution feed line L12 are connected to each other through a sterile connector 73, and thus, it is possible to easily maintain a sterile state after the enzyme solution feed line L12, at the time of replacing the enzyme solution storage tank 71. The enzyme solution storage tank 71 is provided in the refrigerating unit 35, and is retained at a predetermined temperature (for example, 2°C to 6°C) along with the culture solution storage tank 31. Furthermore, in a case where a temperature suitable for the storage of the enzyme solution F4 is different from the temperature suitable for the storage of the culture solution F1, the enzyme solution storage tank 71 may be retained in the refrigerating unit 35 at a temperature different from that of the culture solution storage tank 31, or may be provided in the other refrigerating unit different from the refrigerating unit 35. The enzyme may be a known medicinal solution used in a cell dispersion. Examples of the enzyme include Accutase, Trypsin-EDTA, Dispase, and Collagenase. In addition, in one aspect, a medicinal agent having a cell dispersion function, may be used instead of the enzyme. Examples of the medicinal agent include an EDTA solution or the like.

The automatic cell culture device 100 further includes an inhibitor storage tank 81 storing a Rho-kinase inhibitor, and an inhibitor feeding pump 82 feeding the Rho-kinase inhibitor to the culture tank 11 from the inhibitor storage tank 81. The inhibitor storage tank 81 is connected to the culture tank 11 through an inhibitor feed line L13, and the Rho-kinase inhibitor stored in the inhibitor storage tank 81, as necessary, is fed to the culture tank 11 through the inhibitor feed line L13, by the inhibitor feeding pump 82. The inhibitor storage tank 81 and the inhibitor feed line L13 are connected to each other through a sterile connector 83, and thus, it is possible to easily maintain a sterile state after the inhibitor feed line L13, at the time of replacing the inhibitor storage tank 81. The inhibitor storage tank 81 is provided in the refrigerating unit 35, and is retained to a predetermined temperature (for example, 2°C to 6°C) along with the culture solution storage tank 31. Furthermore, in a case where a temperature suitable for the storage of the Rho-kinase inhibitor, is different from the temperature suitable for the storage of the culture solution F1, the inhibitor storage tank 81 may be retained in the refrigerating unit 35 at a temperature different from that of the culture solution storage tank 31, or may be provided in the other refrigerating unit different from the refrigerating unit 35.

The cell culture device 1 further includes a medicinal solution storage tank 91 storing a medicinal solution, and a medicinal solution feeding pump 92 feeding the medicinal solution to the culture tank 11 from the medicinal solution storage tank 91. The medicinal solution storage tank 91 is connected to the culture tank 11 through a medicinal solution feed line L14, and the medicinal solution stored in the medicinal solution storage tank 91, as necessary, is fed to the culture tank 11 through the medicinal solution feed line L14, by the medicinal solution feeding pump 92. The medicinal solution storage tank 91 and the medicinal solution feed line L14 are connected to each other through a sterile connector 93, and thus, it is possible to easily maintain a sterile state after the medicinal solution feed line L14, at the time of replacing the medicinal solution storage tank 91. The medicinal solution storage tank 91 is provided in the refrigerating unit 35, and is retained at a predetermined temperature (for example, 2°C to 6°C) along with the culture solution storage tank 31. Furthermore, in a case where a temperature suitable for the storage of the medicinal solution is different from the temperature suitable for the storage of the culture solution F1, the medicinal solution storage tank 91 may be retained in the refrigerating unit 35 at a temperature different from that of the culture solution storage tank 31, or may be provided in the other refrigerating unit different from the refrigerating unit 35. The medicinal solution may be a known medicinal solution used in the cell culture. For example, the medicinal solution may be a medicinal agent relevant to maintenance of undifferentiation of cytokine or the like, for acceleration of differentiation, and for activation, or the like, and examples of the medicinal solution include Activin, b-FGF, SCF, TGF-beta, and the like.

The automatic cell culture device 100 further includes a photographing unit 101, and a liquid feed line L15 for photographing to be connected to the photographing unit 101 by branching from the cell collection line L8, and a liquid feeding pump 102 for photographing, feeding a measurement sample including the cell, to the photographing unit 101, is provided in the liquid feed line L15 for photographing. In addition, a buffer tank 103 storing a photographing buffer F5 is connected to the photographing unit 101. The photographing unit 101 photographs the measurement sample fed by the liquid feeding pump 102 for photographing, and acquires a microscope photograph of the cell included in the measurement sample. The measurement sample after the microscope photograph is acquired, may be discarded from the photographing unit 101, as a waste solution. In addition, the measurement sample after the microscope photograph is acquired, may be fed to the cell collection line L8 through the liquid feed line L15 for photographing, and may be collected to the cell collection vial 43. At this time, the liquid feeding pump 102 for photographing may have a function of feeding the measurement sample to the cell collection line L8 from the photographing unit 101. The photographing unit 101 and the buffer tank 103 are provided in the incubator 21, in order to more accurately observe a cell state in the culture environment, and are retained at a predetermined temperature (for example, 35°C to 38°C).

The cell culture device 1 further includes a control unit 130 as the control unit. The control unit 130 is identical to the control unit 130 in the automatic cell culture device 100, illustrated in FIG. 1. The control unit 130 acquires information of a turbidity to be detected by the turbidity meter 15, controls the circulation pump 12 on the basis of the information of the turbidity, and adjusts a circulation rate of the culture solution F1.

An execution procedure of controlling the circulation rate according to the control unit 130, is not particularly limited, and is suitably changed according to the culture environment or the like, in a range where the circulation pump 12 is controlled on the basis of a detection result of the turbidity meter 15. In addition, the control unit 130 may further acquire information to be measured from a meter other than the turbidity meter 15, or may further control a pump other than the circulation pump 12.

In addition, the control unit 130 may acquire a detection result of the dissolved oxygen meter 16, and may control the microbubble pump 14 on the basis of the acquired detection result. In a case where the amount of dissolved oxygen, detected by the dissolved oxygen meter 16, is greater than the preset value, the control unit 130 may decrease a microbubble generation amount of the microbubble pump 14, and in a case where the amount of dissolved oxygen, detected by the dissolved oxygen meter 16, is less than a defined value, the control unit 130 may increase the microbubble generation amount of the microbubble pump 14, such that a preset value of the amount of dissolved oxygen is maintained.

Further, the control unit 130 may acquire a detection result of the pH meter 17, may determine a change-out rate of the culture solution F1, on the basis of the acquired detection result, and may perform a notification with respect to a user or the replacement of the culture solution F1. In addition, the control unit 130 may control each of the culture solution feeding pump 33, the wash solution feeding pump 52, the first waste solution pump 62, and the second waste solution pump 63. In this case, the control unit 130 is capable of controlling each of the pumps according to the own determination or an instruction of the user, and is capable of executing an operation such as the replacement of the culture solution, or the passage. In addition, the cell culture device 1 may further include a liquid amount detector (not illustrated) detecting a liquid amount of the culture solution F1 in the culture tank 11, and the control unit 130 may acquire a detection result of the liquid amount detector, and may control a liquid feeding amount of the culture solution according to the culture solution feeding pump 33, on the basis of the acquired detection result.

The control unit 130 is capable of controlling the cell collection pump 41. In this case, for example, the control unit 130 is capable of automatically controlling cell collection pump 41 (further, as necessary, the culture solution feeding pump 33, the wash solution feeding pump 52, the first waste solution pump 62, and the second waste solution pump 63), after a culture time set in advance, has elapsed, and is capable of automatically collecting the cell.

The control unit 130 may control the enzyme solution feeding pump 72, and in this case, it is possible to feed the enzyme solution F4 to the culture tank 11, on the basis of the instruction of the user or the preset setting. In addition, the control unit 130 may control the inhibitor feeding pump 82, and in this case, it is possible to feed the Rho-kinase inhibitor to the culture tank 11, on the basis of the instruction of the user or the preset setting. In addition, the control unit 130 may control the medicinal solution feeding pump 92, and in this case, it is possible to feed the medicinal solution to the culture tank 11, on the basis of the instruction of the user or the preset setting. In addition, the control unit 130 may control the liquid feeding pump 102 for photographing, and in this case, it is possible to feed the measurement sample to the photographing unit 101, on the basis of the instruction of the user or the preset setting. Further, the control unit 130 may control the photographing of the microscope photograph according to the photographing unit 101, and in this case, it is possible to automatically acquire the microscope photograph of the cell, on the basis of the instruction of the user or the preset setting.

In the automatic cell culture device 100 described above, the cell is suspended in the culture solution F1, and is cultured while floating in the culture solution F1, according to a balance between the upward flow of the culture solution F1 (the upward flow S2 of FIG 4) and the own weight. The control unit 130 controls the circulation rate of the culture solution F1 according to the circulation pump 12, and thus, it is possible to adjust a balance state between the upward flow of the culture solution F1 and the own weight of the cell, and to suitably retain the cell distribution in the culture tank 11.

In addition, in the automatic cell culture device 100, the turbidity meter 15 detects the turbidity of the culture solution F1 in the culture tank 11, and the control unit 130 controls the circulation rate in the execution procedure described above. Accordingly, even in a case where the cell distribution is changed due to a change in the size of the cell, or the like, in a culture procedure, it is possible to automatically adjust the balance state between the upward flow of the culture solution F1 and the own weight of the cell, according to the control of the control unit 130. For this reason, in the cell culture device 1, it is possible to suitably maintain the cell distribution, and to perform homogeneous culture.

In addition, in the automatic cell culture device 100, the turbidity meter 15 measures the turbidity of the culture solution F1 in the culture tank 11, in a plurality of spots in the vertical direction. Here, in a case where the number of cells to be cultured in the culture tank 11, increases, the turbidity of the culture solution F1 increases. In addition, in a case where the size of the cell to be cultured (or the size of the cell mass) increases, the cell distribution is gradually moved to the lower side in the vertical direction, according to the own weight. According to the turbidity meter 15 described above, it is also possible to monitor such a culture state.

In addition, in the automatic cell culture device 100, the amount of dissolved oxygen of the culture solution F1 is detected by the dissolved oxygen meter 16, and thus, the control unit 130 is capable of controlling the microbubble pump 14, on the basis of the detection result. For this reason, in the cell culture device 1, it is possible to easily perform the cell culture in which the amount of dissolved oxygen is maintained to be constant, according to the control of the control unit 130.

In addition, in the automatic cell culture device 100, the pH of the culture solution F1 is detected by the pH meter 17, and thus, the control unit 130 is capable of determining the change-out rate of the culture solution F1, on the basis of the detection result, and of executing the notification with respect to the user or the replacement of the culture solution F1. In such an automatic cell culture device 100, the change-out rate of the culture solution F1 is automatically determined by the control unit 130, and thus, quality degradation of the culture cell or the like due to the overtime of the change-out rate, is prevented.

In addition, in the automatic cell culture device 100, the control unit 130 is capable of controlling each of the culture solution feeding pump 33, the wash solution feeding pump 52, the first waste solution pump 62, and the second waste solution pump 63. For this reason, in the automatic cell culture device 100, it is possible to automatically perform an operation such as the replacement or the passage of the culture solution F1, according to the determination of the control unit 130 or the instruction of the user.

In addition, in the automatic cell culture device 100, the control unit 130 is capable of controlling each of the enzyme solution feeding pump 72, the inhibitor feeding pump 82, and the medicinal solution feeding pump 92. For this reason, in the automatic cell culture device 100, it is possible to automatically supply the enzyme solution, the Rho-kinase inhibitor, and the medicinal solution to the culture tank 11, at an amount and a timing, set in advance, according to the control of the control unit 130.

Next, the details of the culture unit 10 will be described. As described above, the first circulation line L1 to the third circulation line L3 connecting the culture tank 11, the circulation pump 12, and the oxygen supply tank 13 to each other, are included in the culture unit 10. In addition, in the culture unit 10, a relay line L16 connecting the first circulation line L1 and the second circulation line L2 together, is provided. The relay line L16 is a line to be used at the time of attaching the culture unit 10 to the cell culture device 200 for transport, described below. For this reason, the relay line L16 includes an on-off valve, and the on-off valve is in a closed state, in a state where the culture unit 10 is attached to the automatic cell culture device 100.

Further, in the culture unit 10, a heated culture solution feed line L7 connecting the culture solution heating tank 32 and the culture solution heating tank 32, and the culture tank 11, together, may be included. In FIG. 3, a case where the culture solution heating tank 32 is not included in the culture unit 10, is illustrated.

Each of the units included in the culture unit 10 described above, is partitioned from the other units. That is, the culture unit 10 illustrated in FIG. 3, is contained in a housing or the like. The housing of the culture unit 10 may have an isothermal function as with the incubator 21. However, as illustrated in FIG. 3, there are lines connecting each of the tanks included in the culture unit 10, and the tanks on the automatic cell culture device 100 side, together. Therefore, in the culture unit 10, lines corresponding to the respective lines illustrated in FIG. 3, are provided, and are capable of being connected to the lines extending from the tanks or the like not included in the culture unit 10, through the connector or the like. In FIG. 1, a line connecting the culture unit 10 side and the automatic cell culture device 100 side, together, is schematically illustrated.

Thus, the culture unit 10 removable with respect to the automatic cell culture device 100, includes the culture tank 11, the circulation pump 12 for circulating the culture solution in the culture tank 11, the oxygen supply tank 13, and the first circulation line L1 to the third circulation line L3 connecting them to each other. For this reason, the circulation route of the culture solution with respect to the culture tank 11, is ensured, even in a state where the culture unit 10 is removed from the automatic cell culture device 100. Therefore, the culture solution can be circulated in the culture tank 11, insofar as being a state in which the circulation pump 12 can be driven, that is, insofar as being a state in which power can be supplied.

In addition, in the culture unit 10, in a case where the culture tank 11, and the circulation route of the culture solution with respect to the culture tank 11 are ensured, each of the units not included in the culture unit 10, that is, each of the units included in the tank unit 120 for automatic culture, can be shared with the other culture unit 10. Therefore, in the automatic cell culture device 100, each of the units included in the tank unit 120 for automatic culture, is shared with a portion corresponding to the automatic culture tank unit 120. In the cell culture device of the related art, the respective tanks such as the culture solution storage tank 31 storing the culture solution F1, the wash solution storage tank 51 storing the wash solution F2, the waste solution reserving tank 61 reserving the waste solution F3 collected from the culture unit 10, and the enzyme solution storage tank 71 storing the enzyme solution F4, are individually provided in the culture tank 11. However, in the automatic cell culture device 100, the tanks included in the tank unit 120 for automatic culture, are not individually provided in each of the culture units 10, and thus, it is possible to realize mass culture using the culture unit 10.

As with the automatic cell culture device 100, in a case where the tanks included in the tank unit 120 for automatic culture, are connected to the plurality of culture units 10, the pumps provided between each of the tanks and each of the culture units 10 may be individually provided in line portions that are individually provided in each of the culture units 10, in lines connecting each of the tanks and each of the culture units 10 together. Then, the individually provided pumps are controlled by the control unit 130, and thus, it is possible to perform individual control with respect to each of the culture units 10, and to perform suitable control according to the culture situation of the cell in each of the culture units 10. However, the design of the line, the alignment of the pump, or the like, is not limited to the configuration described above, and can be suitably changed.

In a case where the cell is cultured by using the plurality of culture units 10 and by using the automatic cell culture device 100, the culture situation of the cell is changed for each of the culture units 10 (the culture tanks 11). For this reason, for example, a situation is obtained in which the result measured by the turbidity meter 15, the dissolved oxygen meter 16 or the like is different for each of the culture units 10, and thus, there is a case where it is necessary to perform controls different from each other (for example, the replacement with respect to a new medium) with respect to each of the culture units 10, according to the control unit 130. For this reason, in the control unit 130, the information relevant to the culture of the cell is individually acquired with respect to each of the culture units 10 containing in the container 110, and various controls are performed on the basis of the information. In addition, the information acquired by the control unit 130, and the information relevant to the adjustment of the culture environment of the control unit 130, or the like, can be stored in association with the identification tag T attached to each of the culture units 10. Such information may be directly stored with respect to the identification tag T. In this case, the identification tag T itself functions as the storage unit. In addition, only the information for specifying the culture unit 10 may be retained in the identification tag T itself, and the information acquired by the control unit 130, the information relevant to the adjustment of the culture environment of the control unit 130, or the like, may be stored in the external storage device or the like, in association with the information for specifying the culture unit 10. In this case, the external storage device is the storage unit according to this embodiment. In a case where the information relevant to the culture of the cell is stored in the external storage device, the culture unit 10 can be specified by using the information stored in the identification tag T, and thus, the information relevant to the culture unit 10 can be specified and acquired from the information stored in the external storage device. Furthermore, the storage unit may be provided in the automatic cell culture device 100 or the cell culture device 200 for transport, instead of the external storage device.

### (Cell Culture Device for Transport)

Next, the cell culture device 200 for transport will be described with reference to FIG. 5. As described above, in the culture unit 10, the circulation line of the culture solution with respect to the culture tank 11 is ensured even in a state of being removed from the automatic cell culture device 100, and thus, the culture of the cell can be continued while circulating the culture solution F1 in the culture tank 11, insofar as being a state in which the circulation pump 12 can be driven, that is, insofar as being a state in which the power can be supplied. Therefore, the cell culture device 200 for transport, may control the circulation of the culture solution F1 in the culture tank 11 by driving at least the circulation pump 12.

However, in a case where the culture of the cell is continued, it is considered that the culture environment is changed from a preferred culture environment, due to a decrease in an oxygen concentration of the culture solution F1, a temperature change in the culture tank 11, or the like. Therefore, the cell culture system 1 according to this embodiment, includes the cell culture device 200 for transport, enabling the culture of the cell to be continued in the culture unit 10.

FIG. 5 illustrates a state where the culture unit 10 is contained in the container 210 of the cell culture device 200 for transport, and the culture unit 10, and each of the units of the tank unit 220 for transport in the cell culture device 200 for transport, are connected to each other through the lines. In addition, in FIG. 5, the control unit 230 of the cell culture device 200 for transport, is also illustrated. That is, in each of the units included in the cell culture device 200 for transport, illustrated in FIG. 5, each of the units except for the control unit 130, is included in the tank unit 220 for transport, and corresponds to the second culture environment adjustment unit in the cell culture device 100 for transport.

As illustrated in FIG. 5, the culture unit 10 is contained in the container 210 of the cell culture device 200 for transport. The container 210, for example, may function as the incubator. In this case, the inside of the container 210 is adjusted and maintained to a predetermined culture environment (for example, a temperature of 35°C to 38°C, and a carbon dioxide concentration of 5%), and thus, it is possible to prevent the culture environment from varying according to the external environment.

The culture unit 10 contained in the container 210, is capable of circulating the culture solution along a circulation flow path through the oxygen supply tank 13, as with the state illustrated in FIG. 3, that is, a state of being attached to the automatic cell culture device 100. However, in a case where a transport time is prolonged, the amount of dissolved oxygen in the culture solution F1, decreases, and thus, it is considered that the culture environment is degraded. Therefore, in FIG. 5, an example in which the oxygen supply tank 13 is used as the culture solution collection tank while using the lines included in the culture unit 10, is illustrated. In the example illustrated in FIG. 5, the oxygen supply tank 13 is used as the culture solution collection tank, adn thus, the circulation pump 12 and the second circulation line L2 are separated from the oxygen supply tank 13. Then, a high-concentration oxygen dissolved culture solution bag 240 containing the culture solution in which oxygen is dissolved at a high concentration, is connected to the second circulation line L2. In a case where the culture of the cell is continued during the transport time of the culture unit 10, the high-concentration oxygen dissolved culture solution bag 240 contains the culture solution in which an oxygen amount that is considered to be consumed by the culture, is dissolved. In a case where a bag portion of the high-concentration oxygen dissolved culture solution bag 240 is configured of a material having high flexibility, such as polyethylene, the bag can be downsized, and is easily deformed, and thus, an attachment position of the high-concentration oxygen dissolved culture solution bag 240, or the like can be flexibly changed according to the shape of the culture unit 10, or the like. In the configuration described above, the high-concentration oxygen dissolved culture solution bag 240 functions as the second culture solution supply unit supplying the culture solution to the culture tank 11.

In addition, in a state where the culture unit 10 is attached to the cell culture device 200 for transport, the on-off valve of the relay line L16 connecting the first circulation line L1 and the second circulation line L2 together, provided in the culture unit 10, is set in an opened state. Accordingly, the circulation route of the culture solution F1 is configured by the first circulation line L1, the relay line L16, and the third circulation line L3.

In such a state, in a case where the circulation pump 12 is driven, the culture solution F1 is circulated on the circulation route, and a part of the culture solution F1 in which oxygen is consumed by the culture of the cell, flows into the culture solution collection tank (the oxygen supply tank 13). In addition, the culture solution corresponding to the amount of culture solution that flows into the culture solution collection tank (the oxygen supply tank 13), is supplied from the high-concentration oxygen dissolved culture solution bag 240 through the second circulation line L2. As a result thereof, it is possible to maintain a state in which a decrease in the amount of dissolved oxygen of the culture solution F1 that is circulated on the circulation route, is suppressed.

Furthermore, as described above, the cell culture device 200 for transport may include a culture solution tank 270, instead of a configuration of connecting the high-concentration oxygen dissolved culture solution bag 240. In this case, a culture solution feeding pump 233 and the culture solution tank 270 are connected to an upper end of the heated culture solution feed line L7 to be connected to the culture tank 11 of the culture unit 10, and thus, it is possible to supply the culture solution in a state where oxygen is sufficiently dissolved, to the culture tank 11. In this case, the culture solution tank 270 functions as the second culture solution supply unit supplying the culture solution to the culture tank 11.

Further, in a case where the oxygen supply tank 13 itself is upsized in consideration of the transport time of the culture unit 10, culture unit 10 is attached to the cell culture device 200 for transport, and then, it is not necessary to supply the culture solution from the outside. However, in a case where the transport time is prolonged, it is considered that there is a possibility that a change in the culture environment, such as a decrease in the dissolved oxygen, occurs as the transport time has elapsed. The oxygen supply tank 13 is upsized, and thus, it is possible to minimize a change in the culture environment while it is not necessary to supply the culture solution from the outside.

In addition, the cell culture device 200 for transport includes a cell collection line L28 that is the collection route of collecting the cultured cell from the culture tank 11 of the culture unit 10. In addition, a cell collection pump (not illustrated) may be provided on the cell collection line L28. A sterile connector 242 is provided in a connection portion of the cell collection line L28 with respect to the culture unit 10, and thus, the cell collection line L28 can be sterilely connected to a cell collection vial 243. In the cell culture device 200 for transport, the cell collection vial 243 is connected to the sterile connector 42, and then, the cell is circulated through the cell collection line L28 from the culture tank 11, according to the driving of the cell collection pump, or the like, and thus, the cultured cell can be collected. Each of the units described above functions as the second cell collection unit in the cell culture device 200 for transport.

In addition, in a case where the container 210 does not function as the incubator, a warm gas generation unit 250 and a pressure control unit 260 for adjusting and maintaining the inside of the container 210 to a predetermined culture environment, are provided in the container 210.

The warm gas generation unit 250 has a function of supplying gas for setting the inside of the container 210 to be a predetermined culture temperature, into the container 210. Furthermore, the component of the gas can be suitably selected according to the culture environment.

In addition, the pressure control unit 260 has a function of adjusting a gas component to a predetermined culture environment (for example, a carbon dioxide concentration of 5%) by supplying carbon dioxide into the container 210. Further, the pressure control unit 260 may have a function of pressurizing the inside of the container 210 such that the inside of the container 210 has a positive pressure with respect to the external environment. The inside of the container 210 is set to a positive pressure with respect to the external environment, and thus, it is possible to prevent dust, bacteria, or the like from flowing into the container 210 from the outside, and to maintain a clean level of the inside of the container 210. Furthermore, the pressure control unit 260 may function as the warm gas generation unit 250. In this case, the warm gas generation unit 250 may not be provided.

As with the automatic cell culture device 100, a turbidity meter 215 measuring the turbidity of the culture solution F1 in the culture tank 11 in the culture unit 10, at plurality of spots in the vertical direction, is provided in the cell culture device 200 for transport. In the cell culture device 200 for transport, the cell distribution of the culture solution F1 in the culture tank 11 is detected by the turbidity meter 215 as the second cell distribution detection unit.

In addition, the cell culture device 200 for transport includes a dissolved oxygen meter 216 detecting the amount of dissolved oxygen of the culture solution F1 in the culture tank 11. The dissolved oxygen meter 216 may detect the amount of dissolved oxygen before the culture is started, may constantly detect the amount of dissolved oxygen during the culture, or may detect the amount of dissolved oxygen at a predetermined interval during the culture.

Further, the cell culture device 200 for transport further includes a pH meter 217 detecting the pH of the culture solution F1 in the culture tank 11. The pH meter 217 may detect the pH before the culture is started, may constantly detect the pH during the culture, or may detect the pH at a predetermined interval during the culture.

In addition, the cell culture device 200 for transport may include a thermometer 222 measuring the temperature in the culture tank 11 or the temperature of the culture solution F1 in the culture tank 11. In this case, it is possible to confirm that the inside of the culture tank 11 is adjusted and maintained to a predetermined culture environment by the thermometer 222. Furthermore, in the cell culture device 200 for transport, the dissolved oxygen meter 216, the pH meter 217, and the thermometer 222 function as the second culture environment detection unit detecting the culture environment, but a means detecting the culture environment, other than the above description, may be provided.

The control unit 230 of the cell culture device 200 for transport, described above, controls each of the units of the cell culture device 200 for transport, described above.

Specifically, the control unit 230 acquires information of the turbidity to be detected by the turbidity meter 215, controls the circulation pump 12 on the basis of the information of the turbidity, and adjusts the circulation rate of the culture solution F1. In addition, the control unit 230 may acquire a detection result of the dissolved oxygen meter 216, and a supply amount of the culture solution to be supplied from the high-concentration oxygen dissolved culture solution bag 240, or the like, may be adjusted such that a preset value of the amount of dissolved oxygen is maintained. In addition, the control unit 230 may acquire a detection result of the pH meter 17, may determine the change-out rate of the culture solution F1, on the basis of the acquired detection result, and may perform a notification with respect to the user.

The control unit 230 may control the warm gas generation unit 250 and the pressure control unit 260. When such control is performed, for example, temperature information detected by the thermometer 222 is used, and thus, the control can be performed on the basis of the result.

In a case where the cell collection pump is provided in front of the cell collection vial 243, the control unit 230 is capable of controlling the cell collection pump. In this case, for example, the control unit 230 is capable of automatically collecting the cell, by automatically controlling the cell collection pump after the culture time set in advance, has elapsed.

Furthermore, the control unit 230 may control the culture environment of the culture unit 10 by using the identification tag T of the culture unit 10 attached to the cell culture device 200 for transport. As described above, the information for specifying the culture unit 10 can be associated with the identification tag T, and the information relevant to the cell culture of the culture unit 10 can be acquired. Therefore, in the control unit 130 of the cell culture device 200 for transport, information relevant to the past culture environment of the culture unit 10, or the like is acquired by using the identification tag T, and thus, the control relevant to the culture of the cell can be performed on the basis of the information.

In the cell culture device 200 for transport, described above, the cell is suspended in the culture solution F1 in the culture tank 11, and is cultured while floating in the culture solution F1, according to a balance between the upward flow of the culture solution F1 (the upward flow S2 of FIG. 4) and the own weight. The control unit 230 controls the circulation rate of the culture solution F1 according to the circulation pump 12, and thus, it is possible to adjust a balance state between the upward flow of the culture solution F1 and the own weight of the cell, and to suitably retain the cell distribution in the culture tank 11. Therefore, it is possible to preferably culture the cell while the culture unit 10 is transported.

In addition, in the cell culture device 200 for transport, the turbidity meter 215 detects the turbidity of the culture solution F1 in the culture tank 11, and the control unit 230 controls the circulation rate in the execution procedure described above. Accordingly, even in a case where the cell distribution is changed due to a change in the size of the cell, or the like, in the culture procedure, it is possible to automatically adjust the balance state between the upward flow of the culture solution F1 and the own weight of the cell, according to the control of the control unit 230. For this reason, in the cell culture device 200 for transport, it is possible to suitably maintain the cell distribution, and to perform homogeneous culture. In addition, according to the turbidity meter 215 described above, it is also possible to monitor such a culture state. Such a point is the same as the automatic cell culture device 100.

In addition, in the cell culture device 200 for transport, it is possible to detect the information relevant to the culture environment of the culture solution F1, by the dissolved oxygen meter 216, the pH meter 217, the thermometer 222, and the like. Then, the control unit 230 is capable of performing control such that the culture environment is appropriate, on the basis of the detection result. Therefore, according to the cell culture device 200 for transport, it is possible to suitably maintain the culture environment, while the culture unit 10 is transported.

Further, in the cell culture device 200 for transport, the information relevant to the cell culture in the culture unit 10, can be acquired by using the identification tag T attached to the culture unit 10, and the culture environment can be controlled by using the information. Thus, the identification tag T is attached to the culture unit 10, adn thus, the information relevant to the cell culture in the culture unit 10 is easily collected and accumulated, and thus, for example, traceability is easily ensured.

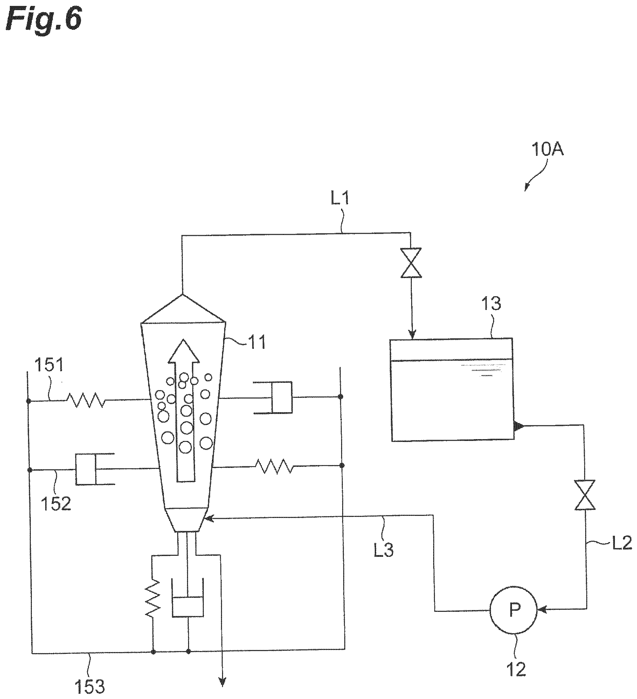

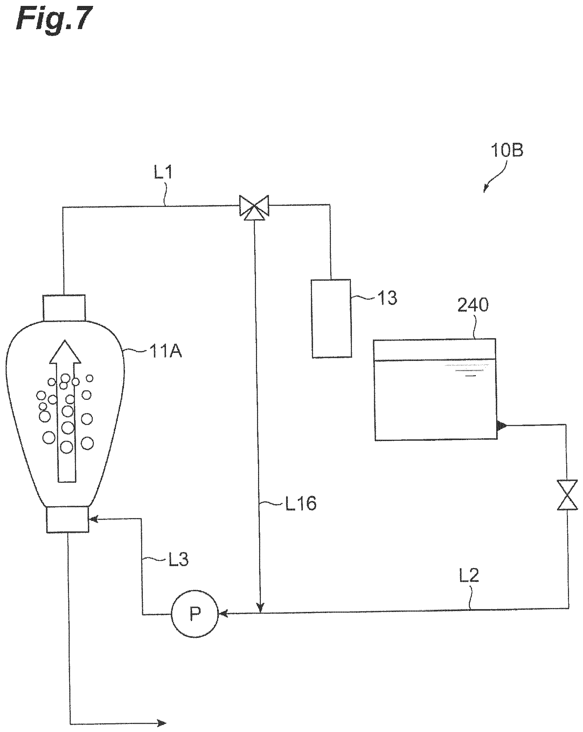

Furthermore, the cell culture device 200 for transport is a device used for transport the culture unit 10, as described above. When the cell culture device 200 for transport is transported, there is a possibility that a considerable vibration occurs. For this reason, when the cell culture device 200 for transport is transported, it is desirable to set a state in which a vibration does not occur in the device itself. However, it is also considered that the vibration of the culture tank 11, which has a possibility of having an influence, is particularly suppressed, instead of suppressing the vibration of the entire device. Hereinafter, a modification example for particularly suppressing the vibration of the culture tank 11, in the culture unit 10 during the transport, will be described with reference to FIG. 6 and FIG. 7. Furthermore, the shape of the circulation route of the culture solution is different from that of FIG. 6 and FIG. 7, but as described above, the circulation route can be suitably changed.

FIG. 6 is a diagram schematically illustrating a culture unit 10A according to a first modification example. In the culture unit 10A, a spring 151, a damper 152, and the like, as the vibration suppression unit, are provided with respect to the culture tank 11, in order to suppress the vibration of the culture tank 11 due to the transport. The spring 151 and the damper 152 are provided to connect a culture tank storage vessel 153 provided around the culture tank 11, and the culture tank 11 together. The culture unit 10A has such a configuration, and thus, not only when the culture unit 10 is transported, but also when the culture unit 10 is removed from or attached the automatic cell culture device 100 or when the culture unit 10, it is possible to further absorb the vibration by the spring 151 and the damper 152, and therefore, it is possible to suppress the vibration of the culture tank 11.

FIG. 7 is a diagram schematically illustrating a culture unit 10B according to a second modification example. In the culture unit 10B, a culture tank 11A itself is different from the other culture tanks 11. Specifically, a vessel portion in the center of the culture tank 11A is configured of a material having flexibility, such as polyethylene, and thus, an external vibration or the like is absorbed by deforming the vessel portion. A material with a high strength is used in both of an upper end and a lower end of the culture tank 11A culture tank 11, and the upper end and the lower end are connected to each other through a circulation line or the like, and thus, the shape of the culture tank 11A is maintained. Furthermore, the vessel portion of the culture tank 11A may have a self-standable thickness. The culture tank 11A of the culture unit 10B has such a configuration, and thus, the vibration of the culture tank 11A, or the like, can be moderated in the vessel portion. Therefore, it is possible to prevent the vibration of the cell in the culture tank 11.

As described above, according to the cell culture system 1 of this embodiment, and the culture units 10, 10A, and 10B, the automatic cell culture device 100, and the cell culture device 200 for transport, included in the cell culture system 1, in a state where the culture unit 10 is contained in the container 110 of the automatic cell culture device 100, the circulation of the culture solution F1 on the circulation route in the culture unit 10 is controlled by the control unit 130, and in a state where the upward flow of the culture solution F1 is formed in the culture tank 11, the cell is cultured. At this time, the culture solution is supplied from the culture solution tank 31 functioning as the first culture solution supply unit, and the cultured cell is collected by the cell collection vial 43 functioning as the first cell collection unit, and thus, the cell culture can be automatically performed. On the other hand, in a state where the culture unit 10 is contained in the container 210 of the cell culture device 200 for transport, the circulation of the culture solution F1 on the circulation route in the culture unit 10 is controlled by the control unit 230. Accordingly, in a state where the upward flow of the culture solution F1 is formed in the culture tank 11, the cell is cultured. For this reason, even in a state where the culture unit 10 is attached to the cell culture device 200 for transport, the circulation of the culture solution F1 is controlled, and thus, the culture of the cell is continued. Therefore, in the automatic cell culture device 100, it is possible to efficiently perform the cell culture, and to perform the transport while suppressing the quality degradation by the cell culture device 200 for transport at the time of performing the transport.

In addition, it is possible to include the identification tag T to which the identification information for specifying the culture unit 10 is individually applied, and to store the information relevant to the cell culture in the storage unit, in association with the identification information. Therefore, in the control unit 130 of the automatic cell culture device 100 and the control unit 230 of the cell culture device 200 for transport, it is possible to acquire the information relevant to the culture of the cell in the culture unit 10, from the storage unit by using the identification information of the identification tag T, and to perform the control using the information, and thus, it is possible to more suitably perform the control relevant to the cell culture of the culture unit 10, for each of the culture units 10.

In addition, as with the culture units 10A and 10B described above, in the case of including the vibration suppression unit for suppressing the vibration of the culture tank 11, it is possible to prevent a vibration at the time of performing the transport by attaching the culture units 10A and 10B to the cell culture device for transport culture tank, from affecting the cell culture.

In addition, in the automatic cell culture device 100 according to this embodiment, the supply amount per unit time of the culture solution F1 to be supplied to the culture tank, is controlled on the basis of the cell distribution in the culture tank 11, detected by the turbidity meter 15 as the first cell distribution detection unit. Accordingly, in a case where the cell distribution in the culture tank F1 is changed from the assumed situation, it is possible to automatically control the supply amount per unit time of the culture solution F1 in order to correct the change. Therefore, it is possible to more preferably control the supply amount of the culture solution according to the growth of the cell, while stabilizing the flow of the culture solution in the culture tank 11.

In addition, in the automatic cell culture device 100 according to this embodiment, the adjustment of the culture solution to be supplied to the culture tank 11 is controlled on the basis of the culture environment in the culture tank 11, detected by the dissolved oxygen meter 16, the pH meter 17, and the thermometer 22, as the first culture environment detection unit, and thus, it is possible to automatically control the adjustment for improving the culture environment in the culture tank 11. Therefore, it is possible to preferably maintain the culture environment according to the growth of the cell in the culture tank 11.

In addition, as with the cell culture device 200 for transport according to this embodiment, the second culture environment adjustment unit including the high-concentration oxygen dissolved culture solution bag 240 or the culture solution tank 270, corresponding to the second culture solution supply unit, and the cell collection vial 243 corresponding to the second cell collection unit, is provided. In this case, even in a case where the transport of the culture unit 10 including the culture tank 11 according to the cell culture device 200 for transport, is prolonged, it is possible to prevent the culture solution F1 from being degraded as the culture period is prolonged, and to preferably collect the cultured cell. Therefore, it is possible to preferably transport the culture unit 10 using the cell culture device 200 for transport.

In addition, in the cell culture device 200 for transport according to this embodiment, the supply amount per unit time of the culture solution F1 to be supplied to the culture tank, is controlled on the basis of the cell distribution in the culture tank 11, detected by the turbidity meter 215 as the second cell distribution detection unit. Accordingly, in a case where the cell distribution in the culture tank F1 is changed from the assumed situation, it is possible to automatically control the supply amount per unit time of the culture solution F1 in order to correct the change. Therefore, it is possible to more preferably control the supply amount of the culture solution according to the growth of the cell, while stabilizing the flow of the culture solution in the culture tank 11.

In addition, in the cell culture device 200 for transport according to this embodiment, the adjustment of the culture solution to be supplied to the culture tank 11, is controlled on the basis of the culture environment in the culture tank 11, detected by the dissolved oxygen meter 216, the pH meter 217, and the thermometer 222, as the second culture environment detection unit, and thus, it is possible to automatically control the adjustment for improving the culture environment in the culture tank 11. Therefore, it is possible to preferably maintain the culture environment according to the growth of the cell in the culture tank 11.

The embodiment as described above, represents one example of the present disclosure. The cell culture system, the culture unit, the automatic cell culture device, and the cell culture device for transport, according to the present disclosure, are not limited to the embodiment described above, and may be modified within a range not changing the gist described in each claim, or may be applied to other objects.

For example, the culture unit 10 may have a part of the function of the automatic cell culture device 100 or the cell culture device 200 for transport, described in the embodiment. For example, the automatic cell culture device 100 includes the incubator 21, and the cell culture device 200 for transport includes the warm gas generation unit 250. On the other hand, a temperature control function is provided on the culture unit 10 side, and thus, a part of the function on the automatic cell culture device 100 or the cell culture device 200 for transport side, may be omitted.

### Industrial Applicability

According to the present disclosure, it is possible to perform transport with suppressing the degradation in quality, while efficiently performing cell culture.

### Reference Signs List

1: cell culture system, 10: culture tank, 11: culture tank, 12: circulation pump, 13: oxygen supply tank, 14: microbubble pump, 15: turbidity meter, 16: dissolved oxygen meter, 17: pH meter, 21: incubator, 22: thermometer, 31: culture solution storage tank, 32: culture solution heating tank, 33: culture solution feeding pump, 34: sterile connector, 35: refrigerating unit, 41: cell collection pump, 42: sterile connector, 43: cell collection vial, 51: wash solution storage tank, 52: wash solution feeding pump, 53: sterile connector, 61: waste solution reserving tank, 62: first waste solution pump, 63: second waste solution pump, 64: sterile connector, 71: enzyme solution storage tank, 72: enzyme solution feeding pump, 73: sterile connector, 81: inhibitor storage tank, 82: inhibitor feeding pump, 83: sterile connector, 91: medicinal solution storage tank, 92: medicinal solution feeding pump, 93: sterile connector, 100: automatic cell culture device, 101: photographing unit, 102: liquid feeding pump for photographing, 103: buffer tank, 110: container, 120: tank unit for automatic culture, 130: control unit, 200: cell culture device for transport, 210: container, 215: turbidity meter, 216: dissolved oxygen meter, 217: pH meter, 220: tank unit for transport, 222: thermometer, 230: control unit, F1: culture solution, F2: wash solution, F3: waste solution, F4: enzyme solution, F5: photographing buffer, L1: first circulation line, L2: second circulation line, L3: third circulation line.

## Claims

1. A cell culture system, comprising:
a culture unit including a culture tank for culturing a cell in a culture solution;
an automatic cell culture device automatically controlling the culture of the cell in the culture unit; and
a cell culture device for transport, controlling the culture of the cell in the culture unit at the time of transporting the culture unit,
wherein the culture unit includes,
the culture tank in a shape in which a horizontal sectional area upwardly increases, and
a circulation route for circulating the culture solution such that the culture solution forms an upward flow in the culture tank,
the automatic cell culture device includes,
a first container capable of containing the culture unit,
a first culture environment adjustment unit including a first culture solution supply unit supplying the culture solution to the culture tank of the culture unit contained in the first container, and a first cell collection unit collecting the cell in the culture tank, and adjusting a culture environment in the culture tank of the culture unit contained in the first container, and
a first control unit controlling the circulation of the culture solution along the circulation route of the culture unit, and controlling the adjustment of the culture environment of the first culture environment adjustment unit, and
the cell culture device for transport includes,
a second container capable of containing the culture unit, and
a second control unit controlling the circulation of the culture solution along the circulation route of the culture unit contained in the second container.

2. The cell culture system according to claim 1,
wherein the culture unit includes identification information for specifying the culture unit, which is individually applied,
the cell culture system further comprises: a storage unit storing information relevant to the culture of the cell in the culture unit, in association with the identification information, and
the first control unit of the automatic cell culture device and the second control unit of the cell culture device for transport respectively acquire the information relevant to the culture of the cell in the culture unit, from the storage unit, on the basis of the identification information, and perform control relevant to the culture unit, on the basis of the information.

3. A culture unit provided in a cell culture system including a culture unit including a culture tank for culturing a cell in a culture solution, an automatic cell culture device automatically controlling the culture of the cell in the culture unit, and a cell culture device for transport, controlling the culture of the cell in the culture unit at the time of transporting the culture unit, the unit comprising:
the culture tank in a shape in which a horizontal sectional area upwardly increases; and
a circulation route for circulating the culture solution such that the culture solution forms an upward flow in the culture tank,
wherein the culture unit is removable with respect to the automatic cell culture device and the cell culture device for transport.

4. The culture unit according to claim 3, further comprising:
a vibration suppression unit for suppressing a vibration of the culture tank.

5. An automatic cell culture device provided in a cell culture system including a culture unit including a culture tank for culturing a cell in a culture solution, an automatic cell culture device automatically controlling the culture of the cell in the culture unit, and a cell culture device for transport, controlling the culture of the cell in the culture unit at the time of transporting the culture unit, the device comprising:
a first container capable of containing the culture unit including the culture tank in a shape in which a horizontal sectional area upwardly increases, and a circulation route for circulating the culture solution such that the culture solution forms an upward flow in the culture tank;
a first culture environment adjustment unit including a first culture solution supply unit supplying the culture solution to the culture tank of the culture unit contained in the first container, and a first cell collection unit collecting the cell in the culture tank, and adjusting a culture environment in the culture tank of the culture unit contained in the first container; and
a first control unit controlling the circulation of the culture solution along the circulation route of the culture unit contained in the first container, and controlling the adjustment of the culture environment of the first culture environment adjustment unit.

6. The automatic cell culture device according to claim 5, further comprising:
a first cell distribution detection unit detecting a distribution of the cell in the culture tank of the culture unit contained in the first container,
wherein the first control unit controls a supply amount per unit time of the culture solution to be supplied to the culture tank by the first culture solution supply unit, on the basis of the distribution of the cell in the culture tank, detected by the first cell distribution detection unit.

7. The automatic cell culture device according to claim 5 or 6, further comprising:
a first culture environment detection unit detecting the culture environment in the culture tank of the culture unit contained in the first container,
wherein the first control unit controls the adjustment of the culture environment of the first culture environment adjustment unit, on the basis of the culture environment in the culture tank, detected by the first culture environment detection unit.

8. A cell culture device for transport, provided in a cell culture system including a culture unit including a culture tank for culturing a cell in a culture solution, an automatic cell culture device automatically controlling the culture of the cell in the culture unit, and a cell culture device for transport, controlling the culture of the cell in the culture unit at the time of transporting the culture unit, the device comprising:
a second container capable of containing the culture unit including the culture tank in a shape in which a horizontal sectional area upwardly increases, and a circulation route for circulating the culture solution such that the culture solution forms an upward flow in the culture tank; and
a second control unit controlling the circulation of the culture solution along the circulation route of the culture unit contained in the second container.

9. The cell culture device for transport according to claim 8, further comprising:
a second culture environment adjustment unit including a second culture solution supply unit supplying the culture solution to the culture tank of the culture unit contained in the second container, and a second cell collection unit collecting the cell in the culture tank,
wherein the second control unit controls the second culture environment adjustment unit.

10. The cell culture device for transport according to claim 9, further comprising:
a second cell distribution detection unit detecting a distribution of the cell in the culture tank of the culture unit contained in the second container,
wherein the second control unit controls a supply amount per unit time of the culture solution to be supplied to the culture tank by the second culture solution supply unit, on the basis of the distribution of the cell in the culture tank, detected by the second cell distribution detection unit.

11. The cell culture device for transport according to claim 9 or 10, further comprising:
a second culture environment detection unit detecting a culture environment in the culture tank of the culture unit contained in the second container,
wherein the second control unit controls the adjustment of the culture environment of the second culture environment adjustment unit, on the basis of the culture environment in the culture tank, detected by the second culture environment detection unit.
